# EUROPÄISCHE PATENTANMELDUNG

(11) **EP 4 663 638 A1**
(43) Veröffentlichungstag der Anmeldung: **17.12.2025**
(21) Anmeldenummer: 24182268.3
(22) Anmeldetag: 14.06.2024
(51) Int. Cl.: C07D 471/10, C07C 303/04, C08G 18/02

(54) **SPIROZYKLISCHES AMMONIUMSALZ UND VERFAHREN ZU SEINER HERSTELLUNG**

(71) Anmelder: Covestro Deutschland AG, 51373 Leverkusen (DE)
(72) Erfinder: Richter, Frank, 51373 Leverkusen (DE); Kostromine, Serguei, 53913 Swisttal-Buschhoven (DE)
(74) Vertreter: Levpat

(57) **Zusammenfassung**

Die Erfindung betrifft ein spirozyklisches Ammoniumsalz enthaltend mindestens ein spirozyklisches Ammoniumkation und mindestens ein Anion sowie ein Verfahren zur Herstellung des spirozyklischen Ammoniumsalzes. Die Erfindung betrifft ferner die Verwendung eines solchen spirozyklischen Ammoniumsalzes als Katalysator für die Oligomerisierung von monomeren organischen Isocyanaten mit einer NCO-Funktionalität > 1, in der Elektrochemie zur Verbesserung der elektrochemischen Aktivität von Lithiumionenbatterien oder als ionische Flüssigkeit.

## Beschreibung

Die Erfindung betrifft ein spirozyklisches Ammoniumsalz enthaltend mindestens ein spirozyklisches Ammoniumkation und mindestens ein Anion sowie ein Verfahren zur Herstellung des spirozyklischen Ammoniumsalzes. Die Erfindung betrifft ferner die Verwendung eines solchen spirozyklischen Ammoniumsalzes als Katalysator für die Oligomerisierung von monomeren organischen Isocyanaten mit einer NCO-Funktionalität > 1, in der Elektrochemie zur Verbesserung der elektrochemischen Aktivität von Lithiumionenbatterien oder als ionische Flüssigkeit.

Spirozyklische Ammoniumsalze sind in vielerlei Hinsicht wertvolle Reagenzien wie z.B. in elektrochemischen Anwendungen (vgl. EP 1919922 und darin zit. Lit.), als Hilfsmittel bei der Zeolithherstellung (JACS 2007, 129, 9066-9079 und darin zit. Lit.), als thermostabile ionische Flüssigkeiten (Phys. Chem. Chem. Phys., 2016, 18, 3339-3351 und darin zit. Lit.) bzw. als Katalysatoren (WO 2015/124504, CN115417850).

In vielen dieser Anwendungen ist der hohe Schmelzpunkt von Nachteil.

Aufgabe der vorliegenden Erfindung war es daher, im Vergleich zum Stand der Technik niedriger schmelzende spirozyklische Ammoniumsalze zur Verfügung zu stellen.

Erfindungsgemäß wurde die Aufgabe gelöst durch ein spirozyklisches Ammoniumsalz enthaltend mindestens ein spirozyklisches Ammoniumkation und mindestens ein Anion, wobei das spirozyklische Ammoniumkation mindestens ein Ladungs-tragendes Sickstoffatom beinhaltet, welches Teil von zwei voneinander verschiedenen Ringsystemen (I) und (II) ist, wobei die voneinander verschiedenen Ringsysteme (I) und (II) jeweils aus C2-C20 Alkylenketten bestehen, wobei die jeweiligen Alkylenketten optional mit Heteroatomen wie Sauerstoff, Stickstoff oder Schwefel in der Alkylenkette unterbrochen sein können, wobei mindestens eine Alkylenkette der zwei voneinander verschiedenen Ringsysteme (I) und (II) mindestens einen, bevorzugt mindestens zwei, besonders bevorzugt zwei bis vier und ganz besonders bevorzugt zwei oder drei von H verschiedene, exozyklische Substituenten enthält und wobei das Anion ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid ist.

Exozyklische Substituenten sind erfindungsgemäß Substituenten in der Seitenkette bzw. den Seitenketten der Ringsysteme (I) und/oder (II).

In einer Ausführung der Erfindung enthält das spirozyklische Ammoniumsalz genau ein spirozyklisches Ammoniumkation und genau ein Anion.

In einer Ausführung der Erfindung enthalten die jeweiligen Alkylenketten nur Kohlenstoff und Wasserstoff, d.h. die jeweiligen Alkylenketten sind nicht mit Heteroatomen wie Sauerstoff, Stickstoff oder Schwefel, speziell Sauerstoff in der Alkylenkette unterbrochen.

In einer alternativen Ausführung der Erfindung sind die jeweiligen Alkylenketten mit Heteroatomen wie Sauerstoff, Stickstoff oder Schwefel, bevorzugt Sauerstoff in der Alkylenkette unterbrochen, wobei erfindungsgemäß unter "unterbrochen" beispielsweise der Austausch einer CH₂-Gruppe der Alkylenkette durch Sauerstoff zu verstehen ist, wobei formal eine Ethereinheit resultiert.

In einer Ausführung der Erfindung ist der mindestens eine von H verschiedene, exozyklische Substituent eine verzweigte oder unverzweigte Alkylgruppe, eine verzweigte oder unverzweigte Zykloalkylgruppe und/oder eine substituierte oder unsubstituierte Arylgruppe, bevorzugt eine verzweigte oder unverzweigte Alkylgruppe.

In einer Ausführung der Erfindung ist der der mindestens eine von H verschiedene, exozyklische Substituent eine verzweigte oder unverzweigte Alkylgruppe, wobei in einer bevorzugten Ausführungsform die verzweigte oder unverzweigte Alkylgruppe 1 bis 20 C-Atome, bevorzugt 1 bis 10 C-Atome enthält.

In einer Ausführung der Erfindung ist das Ringsystem (I) aus 3 bis 5 Atomen, bevorzugt 4 bis 5 Atomen und besonders bevorzugt aus 5 Atomen aufgebaut, wobei das spirozyklische Ammoniumkation jeweils mitgezählt wird.

In einer bevorzugten Ausführung der Erfindung ist das Ringsystem (I) aus 3 bis 5 Atomen, bevorzugt 4 bis 5 Atomen und besonders bevorzugt aus 5 Atomen aufgebaut und enthält keine von H verschiedene, exozyklische Substituenten.

In einer Ausführung der Erfindung ist das Ringsystem (II) aus 6 bis 8 Atomen, bevorzugt 6 bis 7 Atomen und besonders bevorzugt aus 6 Atomen aufgebaut, wobei das spirozyklische Ammoniumkation jeweils mitgezählt wird.

In einer bevorzugten Ausführung der Erfindung enthält das Ringsystem (II) den mindestens einen, bevorzugt die mindestens zwei, besonders bevorzugt die zwei bis vier und ganz besonders bevorzugt die zwei oder drei von H verschiedenen, exozyklischen Substituenten, wobei die mindestens zwei, bevorzugt die zwei bis vier und besonders bevorzugt die zwei oder drei von H verschiedenen, exozyklischen Substituenten zu einer weiteren Reduktion der Schmelztemperatur der spirozyklischen Ammoniumsalze führen.

In einer besonders bevorzugten Ausführung der Erfindung ist das Ringsystem (II) aus 6 bis 8 Atomen, bevorzugt 6 bis 7 Atomen und besonders bevorzugt aus 6 Atomen aufgebaut und mit zwei bis vier, bevorzugt zwei oder drei von H verschiedenen, exozyklischen Substituenten substituiert, die jeweils eine verzweigte oder eine unverzweigte, bevorzugt eine unverzweigte Alkylgruppe umfassen, wobei die verzweigte oder unverzweigte Alkylgruppe jeweils 1 bis 10, bevorzugt 2 bis 6 C-Atome enthält. Hierbei ist in einer Ausführungsform die berechnete Molmasse einer verzweigten oder einer unverzweigten, bevorzugt einer unverzweigten Alkylgruppe größer, als die der mindestens einen weiteren verzweigten oder einer unverzweigten, bevorzugt einer unverzweigten weiteren Alkylgruppe. Hierbei berechnet sich die Molmasse einer Alkylgruppe aus der Summe der Molmassen der enthaltenen Kohlenstoff- und Wasserstoffatome in der jeweiligen Alkylgruppe, wobei beispielsweise für eine Methylgruppe die Molmasse 15 g/mol und für eine Ethylgruppe die Molmasse 29 g/mol resultiert.

Erfindungsgemäß ist das Anion ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid.

Hierbei ist unter einem Polyfluorid ein Addukt von mehr als einem Äquivalent HF an einer FluoridIonen enthaltenden Verbindung zu verstehen.

In einer Ausführungsform der Erfindung ist das Anion ein Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und bevorzugt Triflat, Bis(trifluormethylsulfonyl)imid, Benzolsulfonat, Dodecylbenzolsulfonat Toluolsulfonat, Fluorid oder Hydrogendifluorid und besonders bevorzugt Bis(trifluormethylsulfonyl)imid, Benzolsulfonat, Dodecylbenzolsulfonat, Toluolsulfonat oder Hydrogendifluorid. Ganz besonders bevorzugt ist das Anion Bis(trifluormethylsulfonyl)imid.

Ein weiterer Gegenstand der Erfindung ist ein Verfahren zur Herstellung eines spirozyklischen Ammoniumsalzes umfassend folgende Schritte:
i) Umsetzung einer N-heterozyklischen Alkylverbindung (A) mit der Ringgröße (II) mit einer α,ω-Dihalogenalkylverbindung (B) unter Bildung eines spirozyklisches Ammoniumhalogenids (C), wobei das spirozyklische Ammoniumkation mindestens ein Ladungs-tragendes Sickstoffatom beinhaltet, welches Teil von zwei voneinander verschiedenen Ringsystemen (I) und (II) ist,
ii) optional Umsetzung des spirozyklisches Ammoniumhalogenids (C) aus Schritt i) mit einer ionischen Verbindung (D) bestehend aus einem Kation (D-1) und einem Anion (D-2), wobei das Anion (D-2) ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid ist,
wobei die N-heterozyklische Alkylverbindung (A) und/oder die α,ω-Dihalogenalkylverbindung (B) mindestens einen, bevorzugt mindestens zwei, besonders bevorzugt zwei bis vier und ganz besonders bevorzugt zwei oder drei von H verschiedene, exozyklische Substituenten enthält.

In einer Ausführungsform des erfindungsgemäßen Verfahrens enthält die N-heterozyklische Alkylverbindung (A) mit dem Ringsystem (II) mindestens einen, bevorzugt mindestens zwei, besonders bevorzugt zwei bis vier und ganz besonders bevorzugt zwei oder drei von H verschiedene, exozyklische Substituenten.

In einer Ausführungsform des erfindungsgemäßen Verfahrens ist der mindestens eine von H verschiedene Substituent der N-heterozyklischen Alkylverbindung (A) mit dem Ringsystem (II) eine verzweigte oder unverzweigte Alkylgruppe, eine verzweigte oder unverzweigte Zykloalkylgruppe und/oder eine substituierte oder unsubstituierte Arylgruppe, bevorzugt eine verzweigte oder unverzweigte Alkylgruppe. In einer bevorzugten Ausführungsform enthält die verzweigte oder unverzweigte Alkylgruppe die N-heterozyklische Alkylverbindung (A) mit dem Ringsystem (II) 1 bis 20 C-Atome, bevorzugt 1 bis 10 C-Atome.

In einer bevorzugten Ausführungsform des erfindungsgemäßen Verfahrens enthält die N-heterozyklische Alkylverbindung (A) mit dem Ringsystem (II) mindestens zwei, besonders bevorzugt zwei bis vier und ganz besonders bevorzugt zwei oder drei verzweigte oder unverzweigte Alkylgruppen, wobei die berechnete Molmasse einer verzweigten oder einer unverzweigten, bevorzugt einer unverzweigten Alkylgruppe größer ist, als die der mindestens einen weiteren verzweigten oder einer unverzweigten, bevorzugt einer unverzweigten weiteren Alkylgruppe.

In einer Ausführungsform des erfindungsgemäßen Verfahrens ist das Ringsystem (II) der N-heterozyklischen Alkylverbindung (A) aus 6 bis 8 Atomen, bevorzugt 6 bis 7 Atomen und besonders bevorzugt aus 6 Atomen aufgebaut, wobei das Stickstoffatom jeweils mitgezählt wird. Hierbei resultieren bei Ringgröße 6 ein substituiertes oder unsubstituiertes Piperidin (Azinan) und bei Ringgröße 7 ein substituiertes oder unsubstituiertes Hexamethylenimin (Azepan). In einer bevorzugten Ausführungsform wird ein Alkylgruppen-substituierte Piperidin (Azinan) und/oder ein Alkylgruppen-substituierte Hexamethylenimin (Azepan), bevorzugt ein Alkylgruppen-substituierte Piperidin (Azinan), als N-heterozyklischen Alkylverbindung (A) verwendet.

In einer besonders bevorzugten Ausführungsform des erfindungsgemäßen Verfahrens ist das Ringsystem (II) der N-heterozyklischen Alkylverbindung (A) aus 6 bis 8 Atomen, bevorzugt 6 bis 7 Atomen und besonders bevorzugt aus 6 Atomen aufgebaut, wobei die Verbindung (A) mindestens zwei, bevorzugt zwei bis vier und besonders bevorzugt zwei oder drei verzweigte oder unverzweigte exozyklische Alkylgruppen enthält, wobei die berechnete Molmasse einer verzweigten oder einer unverzweigten, bevorzugt einer unverzweigten Alkylgruppe größer ist, als die der mindestens einen weiteren verzweigten oder einer unverzweigten, bevorzugt einer unverzweigten weiteren Alkylgruppe.

In einer ganz besonders bevorzugten Ausführungsform des erfindungsgemäßen Verfahrens ist die N-heterozyklische Alkylverbindung (A) ein Alkylgruppen-substituiertes Piperidin (Azinan) und/oder ein Alkylgruppen-substituierte Hexamethylenimin (Azepan), bevorzugt ein Alkylgruppen-substituiertes Piperidin (Azinan) mit je mindestens zwei, bevorzugt zwei bis vier und besonders bevorzugt zwei oder drei verzweigten oder unverzweigten, exozyklischen Alkylgruppen, wobei die berechnete Molmasse einer verzweigten oder einer unverzweigten, bevorzugt einer unverzweigten Alkylgruppe größer ist, als die der mindestens einen weiteren verzweigten oder einer unverzweigten, bevorzugt einer unverzweigten weiteren Alkylgruppe.

In einer Ausführungsform des erfindungsgemäßen Verfahrens ist die substituierte oder unsubstituierte N-heterozyklische Alkylverbindung (A) ein gesättigter N-Heterozyklus, erhältlich bzw. wird erhalten durch Kernhydrierung aus der entsprechenden, substituierten oder unsubstituierten N-heterozyklischen, ungesättigten Verbindung. So ist beispielsweise Piperidin durch Kernhydrierung von Pyridin erhältlich. Auch die erfindungsgemäß bevorzugten N-heterozyklischen Alkylverbindungen (A) mit mindestens einem Alkylsubstituenten sind durch Kernhydrierung der entsprechenden N-heterozyklischen, ungesättigten Verbindung mit den entsprechenden Alkylsubstituenten erhältlich wie beispielsweise 2-Methylpiperidin durch Kernhydrierung von 2-Methylpyridin, wobei in der Regel eine Mischung unterschiedlicher Stereoisomere bei der Hydrierung resultiert.

In einer Ausführungsform des erfindungsgemäßen Verfahrens ist die α,ω-Dihalogenalkylverbindung (B) eine substituierte oder unsubstituierte α,ω-Dichloralkylverbindung, eine substituierte oder unsubstituierte α,ω-Dibromalkylverbindung, oder eine substituierte oder unsubstituierte α,ω-Diiodalkylverbindung, bevorzugt eine substituierte oder unsubstituierte α,ω-Dichloralkylverbindung.

In einer bevorzugten Ausführungsform des erfindungsgemäßen Verfahrens ist die α,ω-Dihalogenalkylverbindung (B) eine Alkylgruppen-substituierte oder unsubstituierte α,ω-Dichloralkylverbindung, wobei die Alkylgruppen der Alkylgruppen-substituierten α,ω-Dichloralkylverbindung exozyklisch sind.

In einer besonders bevorzugten Ausführungsform des erfindungsgemäßen Verfahrens enthält die α,ω-Dihalogenalkylverbindung (B) keine von H verschiedene, exozyklische Substituenten.

In einer bevorzugten Ausführungsform des erfindungsgemäßen Verfahrens umfasst die α,ω-Dihalogenalkylverbindung (B) eine oder mehrere Verbindungen und wird ausgewählt aus der Gruppe bestehend aus 1,4-Dichlorbutan, 1,4-Dibrombutan, 1,4-Diiodbutan, 1,5-Dichlorpentan, 1,5-Dibrompentan, 1,5-Diiodpentan, bevorzugt 1,4-Dichlorbutan und 1,5-Dichlorpentan.

Erfindungsgemäß wird das Ammoniumhalogenid (D) aus Schritt i) im nachfolgenden Schritt ii) mit einer ionischen Verbindung (D) bestehend aus einem Kation (D-1) und einem Anion (D-2) umgesetzt, wobei das Anion (D-2) ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid ist.

Erfindungsgemäß kann als Kation (D-1) ein Proton (H⁺), ein Alkalimetallkation, ein Erdalkalimetallkation oder ein Übergangsmetallkation verwendet werden. Bevorzugt wird Lithium (Li⁺), Natrium (Na⁺) und/oder Kalium (K⁺) als Kation (D-1) verwendet.

Erfindungsgemäß ist das Anion (D-2) ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid.

In Schritt i) erfolgt die Umsetzung einer N-heterozyklischen Alkylverbindung mit der Ringgröße (II) mit einer α,ω-Dihalogenalkylverbindung optional in Gegenwart eines Katalysators (A) unter Bildung eines spirozyklisches Ammoniumhalogenids, wobei das dabei entstehende spirozyklische Ammoniumkation mindestens ein Ladungs-tragendes Sickstoffatom beinhaltet, welches Teil von zwei voneinander verschiedenen Ringsystemen (I) und (II) ist.

In Schritt ii) erfolgt die Umsetzung des spirozyklisches Ammoniumhalogenids aus Schritt i) mit einer ionischen Verbindung bestehend aus einem Kation und einem Anion, wobei das wobei das Anion ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid ist.

Ein weiterer Gegenstand der Erfindung ist die Verwendung des erfindungsgemäßen, spirozyklischen Ammoniumsalzes oder eines spirozyklischen Ammoniumsalzes erhältlich nach dem erfindungsgemäßen Verfahren als Katalysator, bevorzugt als Katalysator für die Oligomerisierung von monomeren organischen Isocyanaten mit einer NCO-Funktionalität > 1, in der Elektrochemie zur Verbesserung der elektrochemischen Aktivität von Lithiumionenbatterien oder als ionische Flüssigkeit, bevorzugt als ionische Flüssigkeit.

### Experimentalteil

### Messmethoden

Schmelzpunktbestimmungen erfolgten mit dem Schmelzpunktapparat Mel-Temp der Fa. Electrothermal an kleinen, in mit Stickstoff überschleierten Kapillaren abgefüllten Proben der jeweiligen Substanz und sind nicht korrigiert.

### Beispiel 1 Herstellung spirozyklischer Ammoniumsalze mit unterschiedlichen Kationen und Anionen (z. T. erfindungsgemäß)

### Schritt i): allgemeine Herstellvorschrift zur Herstellung spirozyklischer Ammoniumchloride am nicht erfindungsgemäßen Beispiel das 5-Azaspiro[4.5] dekan-5-iumchlorides (vgl. auch Tabelle 1, Eintrag 2)

In einem 2 l Vierhalskolben mit Rührer und Intensivkühler wurden 61,7 g (1,1 mol) KOH, 500 ml Wasser (entionisiert) und 139,7 g (1,1 mol) 1,4-Dichlorbutan gemeinsam vorgelegt und unter Rühren auf ca. 100-110°C Ölbadtemperatur erhitzt (schwacher Rückfluß).

Nach Erreichen der o.g. Innentemperatur wurden 85,2 g (1 mol) Piperidin so zügig zugegeben, dass nicht zu heftiger Rückfluss eintrat.

Bereits kurz nach Beginn der Piperidinzugabe setzte Feststoffabscheidung ein. Das Reaktionsgemisch war von Beginn an heterogen (anfangs flüssig-flüssig, dann dreiphasig flüssig-flüssig-fest, dann fest-flüssig), enthielt aber immer genügend flüssige Phase für eine effiziente Durchmischung des Großteils der Reaktionsmasse. Optional kann von Fall zu Fall mehr Wasser verwendet werden.

Nach vollständiger Piperidinzugabe wurde zwei Stunden bei 110 - 120°C Ölbadtemperatur nachgerührt. Der Rückfluss ließ in diesem Zeitraum merklich nach.

Anschließend wurden am Rotationsverdampfer bei sukzessive ansteigender Badtemperatur und sukzessiver, an die Destillatmenge angepasster, Absenkung des Druckes alle bis zum Ende bei 0,1 mbar, 150°C flüchtigen Bestandteile abgetrennt und der nahezu farblose, feste Rückstand in 2-Propanol aufgenommen, unter Rühren am Rückfluß gut homogenisiert, filtriert und der Niederschlag mit siedendem 2-Propanol so lange gewaschen, bis ausschließlich KCl (im Vakuum getrocknet: 73,1 g, 98% d. Th.) verblieb.

Die Lösung des spirozyklischen Ammoniumsalzes, 5-Azaspiro[4.5]dekan-5-niumchlorid, wurde am Rotationsverdampfer auf ca. 60% Feststoff eingeengt, wobei nach Abkühlung auf Zimmertemperatur bereits Kristallisation einsetzte, die im Kühlschrank weiter vervollständigt wurde.

Durch Filtration, weiteres Einengen der anfallenden Mutterlaugen und Verarbeitung wie vorstehend beschrieben, Trocknen der anfallenden, farblosen, gut kristallisierenden Feststoffe im Vakuum (3 h, 150°C, 0,1 mbar) wurden summarisch 149,3 g (85% d. Th.) analysenreines 5-Azaspiro[4.5]dekan-5-niumchlorid erhalten, welches für die weiteren Umsetzungen (Anionenaustausch) zum Einsatz kam.

### Schritt ii): Allgemeine Herstellvorschrift zur Umwandlung des in Schritt ii) erhaltenen Chloride in Salze mit alternativen Anionen, hier beispielhaft beschrieben anhand der Herstellung des 5-Azaspiro[4.5]dekan-5-nium-bis(trifluorsulfonyl)imides (nicht erfindungsgemäß), da Angaben aus der Literatur dazu wesentlich umständlicher erscheinen und zudem auch schlechtere Ergebnisse liefern.

Zu 100 g einer 10%igen methanolischen Lithium bis(trifluorsulfonyl)imid-Lösung (34,8 mmol LiNTf₂) wurden unter Rühren 6,1 g 5-Azaspiro[4.5]dekan-5-niumchlorid gegeben und bei Zimmertemperatur gerührt, bis sich alles gelöst hatte. Anschließend wurde Methanol am Rotationsverdampfer abgezogen und der Rückstand bei 100°C/0,1 mbar getrocknet. Das verbliebene Feststoffgemisch wurde in ca. 20 ml Methylenchlorid aufgeschlämmt, das unlösliche Lithiumchlorid durch Filtration abgetrennt, das Filtrat vom Methylenchlorid am Rotationsverdampfer befreit und bei 100°C/0,1 mbar bis zur Gewichtskonstanz getrocknet. Es verblieben 14,5 g (99,1 % d. Th.) 5-Azaspiro[4.5]dekan-5-nium bis(trifluorsulfonyl)imid als leicht gelblicher Feststoff.

Bei den niedriger schmelzenden, erfindungsgemäßen Bis(trifluorsulfonyl)imidsalzen mit höher substituierten spirozyklischen Kationen kann das Aufschlämmen des primär anfallenden Salzgemisches in Methylenchlorid entfallen und das oberhalb des Schmelzpunktes der erfindungsgemäßen Bis(trifluorsulfonyl)imidsalze fest/flüssige Gemisch unmittelbar filtriert werden. Die Löslichkeit von LiCl in den erfindungsgemäßen Bis(trifluorsulfonyl)imidsalzen ist für die meisten Anwendungen hinreichend niedrig, kann aber bei Bedarf durch die üblichen, aus der Literatur bekannten, Methoden weiter abgesenkt werden.

Die anderen, z.T. erfindungsgemäßen, spirozyklischen Ammoniumsalze wurden in Analogie zu o.g. Herstellvorschrift gewonnen.

Die dafür nötigen, substituierten Piperidinderivate, so nicht kommerziell zugänglich, wurden durch Kernhydrierung der entsprechenden Pyridinderivate gewonnen, hier beispielhaft anhand der **Hydrierung des 4-tert.Butylpyridins zum 4-tert.Butylpiperidin (Isomerengemisch)** beschrieben:
In einem gerührten 31 Hochdruckreaktor (V4A-Stahl) wurde 1 kg (7,4 mol) 4-tert-Butylpyridin in 750ml MethylZyklohexanon vorgelegt und unter Rühren (800 U/min) gelöst und 90g des Katalysators Rh/C (Ladung 5%, Fa. Aldrich Kat.N 206164) zugegeben. Es wurde dreimal mit 5 bar Stickstoff inertisiert. Anschließend wurden 50 bar Wasserstoff aufgedrückt, langsam unter Rühren auf 160°C erwärmt, der Wasserstoffdruck auf 100 bar erhöht und so lange Hydriert, bis kein Wasserstoff mehr aufgenommen wurde. Anschließend wurde nach Abkühlen und Entspannen die erhaltene Reaktionsmischung vom Katalysator abfiltriert und am Rotationsverdampfer komplett vom Lösemittel befreit. Es verblieben 914 g einer leicht gelblichen Flüssigkeit, die im Vakuum destilliert wurde: Ölbad 100-105°C, Kopf 67-69°C bei 3 mbar. Ausbeute: 870,4 g (83,% d.Th.). Die ¹H-NMR-Analyse bestätigte die Struktur des Produktes.

Die nicht kommerziell zugänglichen, mehrfach substituierten Pyridinderivate wurde in Anlehnung an RU 2334739 erhalten, wie hier beispielhaft anhand der **Synthese des 3,5-Diethyl-5-propylpyridins** beschrieben sei, welches nach Hydrierung wie vorstehend beschrieben und Umsetzung zum Spirozyklus gemäß der allgemeinen Herstellvorschrift **1a** die in Tabelle 1, Eintrag 12 gezeigten Spezies (als Stereoisomerengemisch) liefert.

Die Reaktion wurde in einem 4L 4-Hals-Kolben durchgeführt. 1600 ml Butanal wurde vorgelegt und unter Rühren auf 0°C abgekühlt. 400 ml 28%ige wäßrige Ammoniaklösung wurden unter Kühlung und kräftigem Rühren innerhalb von wenigen Minuten zugegeben. Anschließend wurde das Reaktionsgemisch in einen Scheidetrichter überführt und nach Phasentrennung die untere, wässrige Phase abgelassen und verworfen. Die obere, organische Phase wurde zurück in den Reaktionskolben überführt und eine Lösung von 54 g Praseodym(III)nitrat Hexahydrat in 608 ml DMF zugegeben. Das Reaktionsgemisch wurde 24 h bei Zimmertemperatur gerührt und anschließend mit 3 mal 300 ml Diethylether extrahiert. Die vereinigten etherischen Extrakte wurden mit MgSO₄ getrocknet und am Rotationsverdampfer eingeengt. Die verbliebene, viskose, gelb-orange Flüssigkeit wurde anschließend mit Hilfe eines Dünnschichtverdampfers bei 160°C Manteltemperatur und 0,15 mbar Druck destilliert. Das erhaltene gelbe Destillat (711 g, 46% d.Th.) 3,5-Diethyl-5-propylpyridin (Isomerengemisch) wurden anschließend ohne weitere Reinigung hydriert wie weiter oben beschrieben.

Um zu Derivaten zu gelangen, die im durch die Zyklisierungsreaktion neu gebildeten Ringsystem substituiert sind, bietet sich ein vierstufiger Weg ausgehend von Nitroalkanen und Maleinsäureestern an, der nachfolgend am Beispiel der Synthese des **2-Isopropyl-1,4-dichlorbutans** (IUPAC Name: 1-Chlor-3-(chlormethyl)-4-methylpentan), welches z.B. für die Herstellung der Kationen 9 und 10 in Tabelle 1 benötigt wird, beschrieben sei.

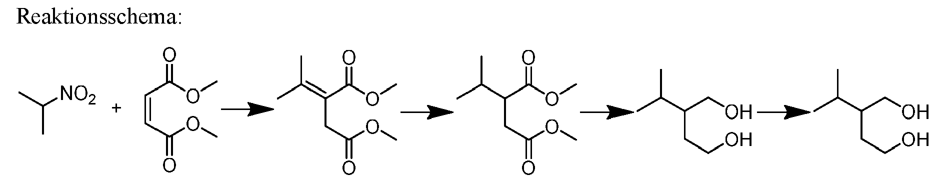

In einem 4l 4-Halskolben wurden 178 g 2-Nitropropan und 288 g Maleinsäuredimethylester in 2500 ml Acetonitril vorgelegt und bei Zimmertemperatur gerührt. 310 g 1,8-DiazabiZyklo[5,4,0]undec-7-en (DBU) wurden bei Zimmertemperatur so zugegeben, dass die Innentemperatur 30°C nicht überstieg. Anschließend wurde bei Zimmertemperatur 7h weitergerührt. In die entstandene, gelbliche, transparente Lösung wurden 500g Kieselgel (Merck 0,04-0,063 mm) eingerührt und abfiltriert. Das Filtrat wurde einrotiert und der verbliebene gelbe Feststoff einer chromatographischen Reinigung unterworfen, Laufmittel: Toluol/Ethanol (2:1). Die das Produkt enthaltende Fraktion wurde am Rotationsverdampfer vom Lösemittel befreit. Es verblieben 367,7 g (98,8% d.Th.) Isopropylidenbemsteinsäuredimethylester als farblose Flüssigkeit (Struktur mittels ¹H- und ¹³C-NMR abgesichert). Letzterer wurde in einem 2l Hydrierreaktor aus V4A-Stahl mit Rührer in 700ml Ethylacetat gelöst vorgelegt und unter Rühren (800 U/min) mit 20 g Katalysator Pd/C (Ladung 10%) versetzt. Nach dreimaliger Inertisierung mit 5 bar Stickstoff und Druckprobe mit 30 bar Stickstoff bei ausgeschaltetem Rührer wurde der Rührer wieder eingesetzt und der Druck auf 5 bar Wasserstoff eingestellt. Anschließend wurde langsam auf 35°C erwärmt und bis zur Beendigung der Wasserstoffaufnahme hydriert. Nach dem Abkühlen wurde entspannt, die Reaktionslösung filtriert und am Rotationsverdampfer vom Lösemittel befreit. Es verblieben 318,5 g einer leicht gelblichen Flüssigkeit, die anschließend im Vakuum destilliert wurde. Ölbad 100-1 15°C° Kopf 55-67°C bei 0,05 -0,1 mbar. Ausbeute an Isopropylbernsteinsäuredimethylester (Struktur mittels ¹H-NMR abgesichert): 300,2 g (81,0% d.Th.).

In einem 4L 4-Hals-Kolben wurden 1200 ml wasserfreier Diethylether vorgelegt, 50 g LiAlH₄ unter Rühren portionsweise zugegeben und gelöst und anschließend eine Lösung von 99,2 g Isopropylbernsteinsäuredimethylester in 400 ml Diethylether langsam zugetropft. Das Reaktionsgemisch wurde 6h bei Zimmertemperatur gerührt und über Nacht stehen gelassen. 1000 ml Wasser wurden langsam und äußerst vorsichtig zugetropft und anschließend mit 20%iger-Salzsäure auf pH 2-3 eingestellt. Das Produkt wurde mit Ethylacetat (2 x 400ml) extrahiert, diese Lösung mit NaSO₄ getrocknet und am Rotationsverdampfer komplett vom Lösemittel befreit. Die verbliebene, farblose Flüssigkeit wurde im Vakuum destilliert. Ölbad 145-150 °C, Kopf:95-97°C bei 0,2mbar. Ausbeute: 65,5 g (94,0% d.Th.) an 2-Isopropyl-1,4-butandiol. Die 1H-NMR-Analyse bestätigte die Struktur.

205 g 2-Isopropyl-1,4-butandiol wurden in einem 4L 4-Hals-Kolben in 205g Pyridin gelöst und 729g Thionylchloirid langsam, unter Außenkühlung zugetropft, wobei die Temperatur der Reaktionsmischung unter 25°C blieb. Es bildete sich zunächst ein weißer Niederschlag der bei der weiteren Thionylchloridzugabe wieder in Lösung ging. Das Reaktionsgemisch wurde 2h bei 100°C (Bad) nachgerührt, dabei klarte die Lösung weiter auf und verfärbte sich zunehmend dunkel. Nach Abkühlung auf Zimmertemperatur wurde eine 1: 1 Mischung aus Eis und Wasser (2330g insgesamt) langsam und äußerst vorsichtig zugegeben und nach vollständigerr Zugabe noch 1h gerührt. Das Produkt wurde mit Diethylether (3 x 300ml) extrahiert und die vereingten Extrakte mit 50%-iger Schwefelsäure und dann mit Natriumhydrogencarbonat-Lösung gewaschen und neutralisiert, mit MgSO₄ getrocknet und am Rotationsverdampfer komplett vom Lösemittel befreit. Nach Vakuumdestillation (Ölbad 115°C, Kopf:44-45°C bei 0,02mbar) resultierten 220,8 g (84,2% d.Th.) lt. ¹H-NMR-Analyse reinen 2-Isopropyl-1,4-dichlorbutans (IUPAC Name: 1-Chlor-3-(chlormethyl)-4-methylpentan).

| Bsp. | Formel des Kations (1-3 : Vergleich, ab 4: erfindungsgemäß) | m.p. Cl⁻ [°C] | m.p. NTf₂⁻ [°C] |
|---|---|---|---|
| 1 (Vgl.) | 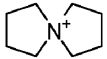 | 289-295⁽¹⁾ | 113-115⁽²⁾ |
| 2 (Vgl.) | 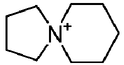 | 290-293 | 110-112⁽²⁾ |
| 3 (Vgl.) | 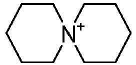 | 300⁽³⁾ | 127-129⁽²⁾ |
| 4 | 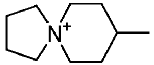 | 233-234 | 62-64 |
| 5 | 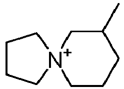 | 285 | 38-40 |
| 6 | 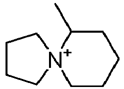 | 238-240 | 62-63 |
| 7 | 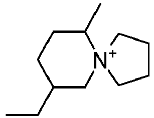 | 157-161 | <25 |
| 8 | 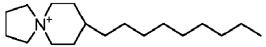 | 293 | 43 - 45 |
| 9 | 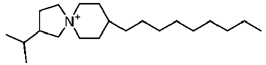 | 330 | <25 |
| 10 | 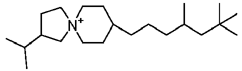 | 330 (z) | <25 |
| 11 | 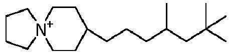 | 278 | <25 |
| **12** | 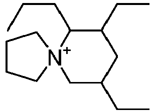 | <25 | <25 |
| **13** | 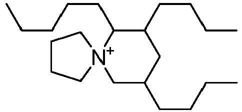 | <25⁽⁴⁾ | <25 |
| **14** | 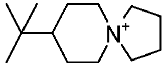 | 335 | 94 |
| **15** | 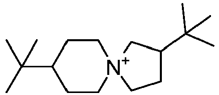 | 320 (Z) | 103 |
| **16** | 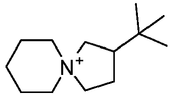 | 325 | 71 |

| | | | |
|---|---|---|---|
| ⁽¹⁾ Bobbitt, J. M.; Amundsen, L.wrence H.; Steiner, Russell I. J. Org. Chem. 1960, 25, 2230 ⁽²⁾ Ward, A. J.; RSC Advances 2014, 4(44), 23327-23337 ⁽³⁾ Kitamura, Masanori; Asian Journal of Organic Chemistry, (2016), 5(12), 1508-1517 ⁽⁴⁾ Hauptteil des Isomerengemisches ist bei r.t. flüssig; etwas Bodensatz isoliert (Bruttozusammensetzung identisch zur flüssigen Isomerenmischung): m.p.: 126-128°C, Das NTf₂-Salz daraus schmilzt jedoch ebenfalls unter 25°C. | | | |

Wie man bei einer vergleichenden Betrachtung der Schmelzpunkte der (nicht erfindungsgemäßen) Chloridsalze im Vergleich zu den erfindungsgemäßen Bis(trifluorsulfonyl)imiden erkennt, ist allein aus dem Schmelzpunkt des (nicht erfindungsgemäßen) Chloridsalzes keine Aussage bezüglich des zu erwartenden Schmelzpunktes des daraus resultierenden, erfindungsgemäßen Bis(trifluorsulfonyl)imides abzuleiten, s. z.B. die Paare 1 und 5: beide Chloride weisen einen ähnlichen Schmelzbereich nahe 300°C auf, während das nicht erfindungsgemäße Bis(trifluorsulfonyl)imid zu 1 deutlich oberhalb 100°C schmilzt und das erfindungsgemäße Bis(trifluorsulfonyl)imid zu 5 bereits bei ca. 40°C flüssig ist

## Patentansprüche

1. Spirozyklisches Ammoniumsalz enthaltend mindestens ein spirozyklisches Ammoniumkation und mindestens ein Anion,
wobei das spirozyklische Ammoniumkation mindestens ein Ladungs-tragendes Sickstoffatom beinhaltet, welches Teil von zwei voneinander verschiedenen Ringsystemen (I) und (II) ist,
wobei die voneinander verschiedenen Ringsysteme (I) und (II) jeweils aus C2-C20 Alkylenketten bestehen,
wobei die jeweiligen Alkylenketten optional mit Heteroatomen wie Sauerstoff, Stickstoff oder Schwefel in der Alkylenkette unterbrochen sein können,
wobei mindestens eine Alkylenkette der zwei voneinander verschiedenen Ringsysteme (I) und (II) mindestens einen, bevorzugt mindestens zwei, besonders bevorzugt zwei bis vier und ganz besonders bevorzugt zwei oder drei von H verschiedene, exozyklische Substituenten enthält,
wobei der mindestens eine, bevorzugt die mindestens zwei, besonders bevorzugt die zwei bis vier und ganz besonders bevorzugt die zwei oder drei von H verschiedene Substituenten über eine Kohlenstoff-Kohlenstoff-Bindung, bevorzugt eine Kohlenstoff-Kohlenstoff-Einfachbindung an mindestens ein Kohlenstoffatom, bevorzugt mindestens zwei voneinander verschiedener Kohlenstoffatome, besonders bevorzugt zwei bis vier voneinander verschiedener Kohlenstoffatome und ganz besonders bevorzugt zwei oder drei voneinander verschiedener Kohlenstoffatome der C2-C20 Alkylenketten des Ringsystems (I) und/oder (II) gebunden ist,
und wobei das Anion ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid ist.

2. Spirozyklisches Ammoniumsalz gemäß Anspruch 1, wobei das spirozyklische Ammoniumsalz genau ein spirozyklisches Ammoniumkation und genau ein Anion enthält.

3. Spirozyklisches Ammoniumsalz gemäß Anspruch 1 oder 2, wobei das Ringsystem (I) aus 3 bis 5 Atomen, bevorzugt 4 bis 5 Atomen und besonders bevorzugt aus 5 Atomen aufgebaut ist, wobei das spirozyklische Ammoniumkation jeweils mitgezählt wird.

4. Spirozyklisches Ammoniumsalz gemäß einem der Ansprüche 1 bis 3, wobei das Ringsystem (II) aus 6 bis 8 Atomen, bevorzugt 6 bis 7 Atomen und besonders bevorzugt aus 6 Atomen aufgebaut ist, wobei das spirozyklische Ammoniumkation jeweils mitgezählt wird.

5. Spirozyklisches Ammoniumsalz gemäß einem der Ansprüche 1 bis 4, wobei die jeweiligen Alkylenketten nur Kohlenstoff und Wasserstoff enthalten.

6. Spirozyklisches Ammoniumsalz gemäß einem der Ansprüche 1 bis 5, wobei der mindestens eine von H verschiedene Substituent eine verzweigte oder unverzweigte Alkylgruppe, eine verzweigte oder unverzweigte Zykloalkylgruppe und/oder eine substituierte oder unsubstituierte Arylgruppe, bevorzugt eine verzweigte oder unverzweigte Alkylgruppe ist.

7. Spirozyklisches Ammoniumsalz gemäß einem der Ansprüche 1 bis 6, wobei der mindestens eine von H verschiedene Substituent eine verzweigte oder unverzweigte Alkylgruppe ist.

8. Spirozyklisches Ammoniumsalz gemäß Anspruch 7, wobei die verzweigte oder unverzweigte Alkylgruppe 1 bis 20 C-Atome, bevorzugt 1 bis 10 C-Atome enthält.

9. Spirozyklisches Ammoniumsalz gemäß einem der Ansprüche 4 bis 8, wobei das Ringsystem (II) den mindestens einen, bevorzugt die mindestens zwei, besonders bevorzugt die zwei bis vier und ganz besonders bevorzugt die zwei oder drei von H verschiedenen, exozyklische Substituenten enthält.

10. Spirozyklisches Ammoniumsalz gemäß einem der Ansprüche 4 bis 9, wobei das Ringsystem (II) aus 6 bis 8 Atomen, bevorzugt 6 bis 7 Atomen und besonders bevorzugt aus 6 Atomen aufgebaut ist und zwei bis vier, bevorzugt zwei oder drei von H verschiedenen, exozyklischen Substituenten mit jeweils einer verzweigten oder einer unverzweigten, bevorzugt einer unverzweigten Alkylgruppe sind, wobei die verzweigte oder unverzweigte Alkylgruppe jeweils 1 bis 10, bevorzugt 2 bis 6 C-Atome enthält.

11. Spirozyklisches Ammoniumsalz gemäß Anspruch 10, wobei die berechnete Molmasse einer verzweigten oder einer unverzweigten, bevorzugt einer unverzweigten Alkylgruppe größer ist, als die der mindestens einen weiteren verzweigten oder einer unverzweigten, bevorzugt einer unverzweigten weiteren Alkylgruppe.

12. Spirozyklisches Ammoniumsalz gemäß Anspruch 3 bis 11, wobei das Ringsystem (I) aus 3 bis 5 Atomen, bevorzugt 4 bis 5 Atomen und besonders bevorzugt aus 5 Atomen aufgebaut, keine von H verschiedenen, exozyklischen Substituenten enthält.

13. Spirozyklisches Ammoniumsalz gemäß einem der Ansprüche 1 bis 12, wobei das Anion Bis(trifluormethylsulfonyl)imid, Benzolsulfonat, Dodecylbenzolsulfonat, Toluolsul-fonat oder Hydrogendifluorid, bevorzugt Bis(trifluormethylsulfonyl)imid ist.

14. Verfahren zur Herstellung eines spirozyklischen Ammoiumsalzes umfassend folgende Schritte:
i) Umsetzung einer N-heterozyklischen Alkylverbindung (A) mit der Ringgröße (II) mit einer α,ω-Dihalogenalkylverbindung (B) unter Bildung eines spirozyklisches Ammoniumhalogenids (C), wobei das spirozyklische Ammoniumkation mindestens ein Ladungs-tragendes Sickstoffatom beinhaltet, welches Teil von zwei voneinander verschiedenen Ringsystemen (I) und (II) ist,
ii) Umsetzung des spirozyklischen Ammoniumhalogenids (C) aus Schritt i) mit einer ionischen Verbindung (D) bestehend aus einem Kation (D-1) und einem Anion (D-2), wobei das wobei das Anion (D-2) ein Perfluoralkylsulfonat, ein Bis(perfluoralkylsulfonyl)imid, ein Alkanoat, ein Carboxylat, ein unsubstituiertes Arylsulfonat, ein am aromatischen Ring substituiertes Arylsulfonat, ein Heterocyclus mit mindestens einem negativ geladenen Stickstoffatom im Ring, ein Fluorid, ein Hydrogendifluorid oder ein höheres Polyfluorid, bevorzugt Triflat, Bis(trifluormethylsulfonyl)imid, Benzolsulfonat, Dodecylbenzolsulfonat, Toluolsulfonat, Fluorid, Hydrogendifluorid, ein Azolat, ein Imidazolat oder ein Triazolat und besonders bevorzugt Triflat, Bis(trifluormethylsulfonyl)imid, Benzolsulfonat, Dodecylbenzolsulfonat Toluolsulfonat, Fluorid oder Hydrogendifluorid und ganz besonders bevorzugt Bis(trifluormethylsulfonyl)imid, Benzolsulfonat, Dodecylbenzolsulfonat, Toluolsulfonat oder Hydrogendifluorid ist,
wobei die N-heterozyklische Alkylverbindung (A) und/oder die α,ω-Dihalogenalkylverbindung (B) mindestens einen, bevorzugt mindestens zwei, besonders bevorzugt zwei bis vier und ganz besonders bevorzugt zwei oder drei von H verschiedene, exozyklische Substituenten enthält.

15. Verwendung des spirozyklischen Ammoniumsalzes gemäß einem der Ansprüche 1 bis 13 oder eines spirozyklischen Ammoniumsalzes erhältlich nach dem Verfahren gemäß Anspruch 14 als Katalysator, bevorzugt als Katalysator für die Oligomerisierung von monomeren organischen Isocyanaten mit einer NCO-Funktionalität > 1, in der Elektrochemie zur Verbesserung der elektrochemischen Aktivität von Lithiumionenbatterien oder als ionische Flüssigkeit.
